# EUROPEAN PATENT SPECIFICATION

(11) **EP 3 061 806 B1**
(45) Date of publication and mention of the grant of the patent: **24.10.2018**
(21) Application number: 16157146.8
(22) Date of filing: 24.02.2016
(51) Int. Cl.: C12M 1/107, C12M 1/00, C12M 1/34

(54) **BIOMASS DIGESTER**
BIOMASSEFERMENTER
DIGESTEUR DE BIOMASSE

(30) Priority: 24.02.2015 NL 1041203; 26.05.2015 NL 2014854
(43) Date of publication of application: 31.08.2016
(73) Proprietor: Host Holding B.V., 7521 PS Enschede (NL)
(72) Inventor: TE BRAAK, Marcel Anton Franciscus, 7577 LM OLDENZAAL (NL)
(74) Representative: Slikker, Wilhelmina Johanna

(56) References cited:
- WO-A1-83/03884
- CN-B- 101 560 466
- US-A- 4 230 580

## Description

The invention relates to a biomass digester, comprising:
- a container for containing a biomass and/or a digestate thereof, and
- a flexible cover for covering the container.
Such a digester is known perse. The flexible cover is provided for closing off said container and thereby preventing or at least reducing the emission of the biomass and/or the digestate thereof. In addition, any biogas generated by the biomass may be stored in the container. WO 83/03884 A describes such a container adapted for the digestion of biomass wherein the flexible cover is provided with a thermal isolation coating layer.

It is an object of the invention to improve the known biomass digester.

This object is achieved by a biomass digester according to the preamble, wherein the cover comprises a thermal reflective layer for reflecting radiation emitted by said biomass and/or digestate.

Said biomass and/or digestate may be heated for improving the digestion thereof, for example between 35 and 55°C. For this purpose, the biomass digester may comprise a heating means for heating the biomass and/or digestate thereof. Alternatively said biomass and/or digestate thereof may be heated prior to being fed to said container, such that relatively warm biomass and/or digestate thereof is contained in said container having no heating means. With the known biomass digester a lot of the heat is lost by radiation emitted by said biomass and/or digestate. This heat loss needs to be compensated by providing additional heating of biomass contained in a container having a heating means or may result in a relatively fast cooling of the biomass in a container having no heating means, thereby reducing the amount of biogas produced. The cover with the thermal reflective layer according to the invention reflects at least partly the radiation emitted by said biomass and/or digestate, thereby reducing the heat loss. As the heat loss is reduced, less heating is required, thereby reducing the amount of energy required for heating the biomass and/or digestate thereof or, if no heating means is provided, the biomass cools down slower such that the production of biomass may be increased in comparison with the container having a cover without the reflective layer according to the invention.

The applicant has found that in particular radiation emitted by said biomass and/or digestate results in a relatively large amount of heat that is lost. Therefore, a relatively large energy saving may be obtained by the cover according to the invention.

In an embodiment of the biomass digester according to the invention said thermal reflective layer has a thermal emissivity of less than 0.1, preferably less than 0.05.

Such a thermal reflective layer efficiently reflects the radiation emitted by the biomass and/or digestate.

In another embodiment of the biomass digester according to the invention said thermal reflective layer is provided on said cover as a coating.

Alternatively, said thermal reflective layer may be said cover.

Practically said thermal reflective layer is arranged on a side of said cover that is facing said container.

In another embodiment of the biomass digester according to the invention, said digester comprises a plurality of flexible covers, wherein at least one cover of said plurality of covers comprises said thermal reflective layer.

If multiple flexible covers are provided, the outer flexible cover may be held upwards in a tight position by air blown in an area defined between the outer flexible cover and a neighboring cover.

Preferably, (a) said cover further comprises a thermal insulating layer. Such a layer reduces the heat loss by convection and may thereby further reduce the heat loss. The thermal insulating layer may for example comprise a (polyurethane) foam.

In yet another embodiment of the biomass digester according to the invention, (a) said cover comprises a synthetic membrane, for example comprising polyvinylchloride (PVC).

Such a membrane comprises a synthetic fabric, sealed with a layer of PVC. Such a membrane is known per se as a cover for a biomass digester.

Optionally said cover or membrane may be provided, in particular coated, with protective or diffusion reducing materials.

The invention further relates to a flexible cover arranged to and intended for covering a container of a biomass digester. In accordance with the invention said cover comprises a thermal reflective layer for reflecting radiation emitted a biomass and/or digestate thereof contained in said container. The advantages of such a reflective layer are described above.

In an embodiment of the cover according to the invention said thermal reflective layer has a thermal emissivity of less than 0.1, preferably less than 0.05.

In another embodiment of the cover according to the invention said thermal reflective layer is provided on said cover as a coating.

In yet another embodiment of the cover according to the invention said thermal reflective layer is said cover.

In yet another embodiment of the cover according to the invention, said cover comprises a thermal insulating layer, wherein said thermal reflective layer is arranged on an outer side of said thermal insulating layer.

In yet another embodiment of the cover according to the invention, said cover comprises a synthetic membrane, for example comprising polyvinylchloride (PVC), wherein said thermal reflective layer is arranged on an outer side of said synthetic membrane, or, where appropriate, on an outer side of said thermal insulating layer.

The invention will be further elucidated with reference to a figure shown in a drawing, in which figure a biomass digester according to an embodiment of the invention is shown schematically in a vertical cross section.

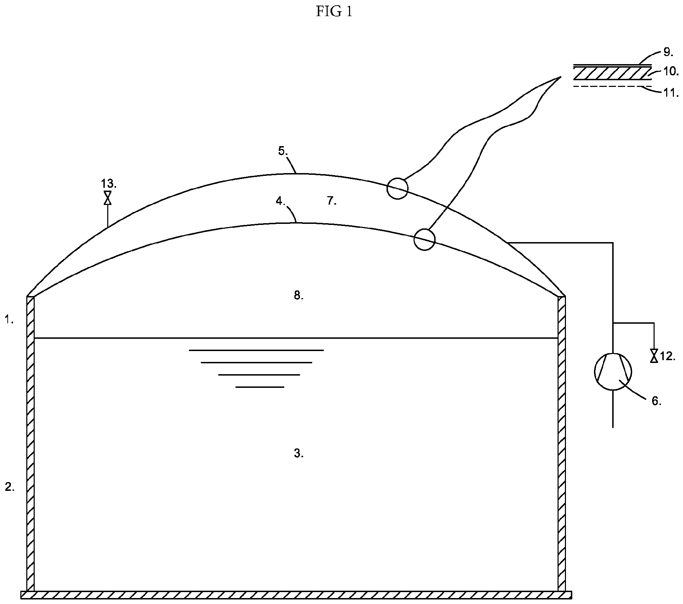

The figure shows a biomass digester 1 comprising a container 2 for containing a biomass 3 and/or a digestate thereof. Said container 2 is closed off by a first, inner flexible cover 4 and a second, outer flexible cover 5. A blower or fan 6 is used for blowing air in an area 7 defined between the two covers 4, 5, such that the outer cover 5 is held tight. A first pressure relief valve 12 is connected to the conduit providing the medium through flow connection between the fan 6 and the area 7, such that any excess air that cannot be contained in the area 7 can be discharged via valve 12. A second pressure relief valves 13 is connected to said area 7 at an opposing end of outer cover 5, in particular near the circumference of the outer cover 5 diametrically with respect to the connection of the conduit of fan 6 to outer cover 5. The amount of air and optionally condense and biogas discharged via valve 13 can be set as desired, such that the ventilation of the air/gas/condense mixture present in area 7 can be provided. By ventilating the area 7 accumulation of condense and/or biogas in the area 7 is prevented. With use of said pressure relief valves 12, 13 and a desired setting thereof it is possible to maintain the amount of air supplied to and maintained in the area 7 at a minimum, thereby reducing the amount of heat lost via convection via the air in area 7 and the amount of heat lost with air discharged from area 7 via said valve 13.

Any biogas generated by the biomass 3 can be stored in an area 8 defined between the level of biomass 3 and the first cover 4. The biomass 3 can optionally be heated by any desired heating means (not shown). Such heating means are known per se. To prevent or reduce the heat loss via said covers 4, 5, said covers 4, 5 are each layered as shown in the detailed view. Each cover 4, 5 comprises a synthetic membrane 9, for example comprising PVC, that is the most outer layer of the cover. A middle layer of each cover 4, 5 is formed by a thermal insulating layer 10, for example comprising a (polyurethane) foam. Said thermal insulating layer 10 prevents or reduces heat loss via convection. An inner layer of each cover 4, 5 comprises a thermal reflective layer 11 that is for example coated on the middle layer 10, in particular on a surface of the middle layer 10 that is facing the container 2. Said layer 11 may for example have a thermal emissivity of less than 0.1, preferably less than 0.05, such that radiation emitted by said biomass and/or digestate is reflected. The covers 4, 5 according to the invention effectively reduce the heat loss by reducing the heat loss via radiation and convection.

It is noted that the invention is not limited to the shown embodiments but also extends to variants within the scope of the appended claims.

## Claims

1. Biomass digester, comprising:
- a container for containing a biomass and/or a digestate thereof, and
- a flexible cover for covering the container;
**characterized in that**
said cover comprises a thermal reflective layer for reflecting radiation emitted by said biomass and/or digestate.

2. Biomass digester according to claim 1, wherein said thermal reflective layer has a thermal emissivity of less than 0.1, preferably less than 0.05.

3. Biomass digester according to claim 1 or 2, wherein said thermal reflective layer is provided on said cover as a coating.

4. Biomass digester according to claim 1 or 2, wherein said thermal reflective layer is said cover.

5. Biomass digester according to any of the preceding claims, wherein said thermal reflective layer is arranged on a side of said cover that is facing said container.

6. Biomass digester according to any of the preceding claims, comprising a plurality of flexible covers, wherein at least one cover of said plurality of covers comprises said thermal reflective layer.

7. Biomass digester according to any of the preceding claims, wherein (a) said cover comprises a thermal insulating layer, and wherein said thermal reflective layer is arranged on an outer side of said thermal insulating layer.

8. Biomass digester according to any of the preceding claims, wherein (a) said cover comprises a synthetic membrane, for example comprising polyvinylchloride (PVC), and wherein said thermal reflective layer is arranged on an outer side of said synthetic membrane, or, where appropriate, on an outer side of said thermal insulating layer.

9. Flexible cover arranged and intended for covering a container of a biomass digester,
**characterized in that**
said cover comprises a thermal reflective layer for reflecting radiation emitted a biomass and/or digestate thereof contained in said container.

10. Flexible cover according to claim 9, wherein said thermal reflective layer has a thermal emissivity of less than 0.1, preferably less than 0.05.

11. Flexible cover according to claim 9 or 10, wherein said thermal reflective layer is provided on said cover as a coating.

12. Flexible cover according to claim 9 or 10, wherein said thermal reflective layer is said cover.

13. Flexible cover according to any of the preceding claims 9 - 12, wherein said cover comprises a thermal insulating layer, and wherein said thermal reflective layer is arranged on an outer side of said thermal insulating layer.

14. Flexible cover according to any of the preceding claims 9 - 13, wherein said cover comprises a synthetic membrane, for example comprising polyvinylchloride (PVC), and wherein said thermal reflective layer is arranged on an outer side of said synthetic membrane, or, where appropriate, on an outer side of said thermal insulating layer.

## Patentansprüche

1. Biomassefermenter, der aufweist:
- einen Behälter zum Aufnehmen einer Biomasse und/oder eines Gärguts daraus, und
- eine flexible Abdeckung zum Abdecken des Behälters;
**dadurch gekennzeichnet, dass**
die Abdeckung eine wärmereflektierende Schicht zum Reflektieren von Strahlung aufweist, die von der Biomasse und/oder dem Gärgut emittiert wird.

2. Biomassefermenter nach Anspruch 1, wobei die wärmereflektierende Schicht ein Wärmeemissionsvermögen von weniger als 0,1, vorzugsweise weniger als 0,05, hat.

3. Biomassefermenter nach Anspruch 1 oder 2, wobei die wärmereflektierende Schicht auf der Abdeckung als eine Beschichtung bereitgestellt ist.

4. Biomassefermenter nach Anspruch 1 oder 2, wobei die wärmereflektierende Schicht die Abdeckung ist.

5. Biomassefermenter nach einem der vorhergehenden Ansprüche, wobei die wärmereflektierende Schicht auf einer Seite der Abdeckung angeordnet ist, die dem Behälter zugewandt ist.

6. Biomassefermenter nach einem der vorhergehenden Ansprüche, der mehrere flexible Abdeckungen aufweist, wobei wenigstens eine Abdeckung der mehreren Abdeckungen die wärmereflektierende Schicht aufweist.

7. Biomassefermenter nach einem der vorhergehenden Ansprüche, wobei (a) die Abdeckung eine wärmeisolierende Schicht aufweist, und wobei die wärmereflektierende Schicht auf einer Außenseite der wärmeisolierenden Schicht angeordnet ist.

8. Biomassefermenter nach einem der vorhergehenden Ansprüche, wobei (a) die Abdeckung eine synthetische Membran aufweist, die zum Beispiel Polyvinylchlorid (PVC) aufweist, und wobei die wärmereflektierende Schicht auf einer Außenseite der synthetischen Membran oder gegebenenfalls auf einer Außenseite der wärmeisolierenden Schicht angeordnet ist.

9. Flexible Abdeckung, die angeordnet und dafür gedacht ist, um einen Behälter eines Biomassefermenters abzudecken, **dadurch gekennzeichnet, dass** die Abdeckung eine wärmereflektierende Schicht zum Reflektieren von Strahlung aufweist, die von der Biomasse und/oder ihrem Gärgut, die in dem Behälter enthalten sind, emittiert wird.

10. Flexible Abdeckung nach Anspruch 9, wobei die wärmereflektierende Schicht ein Wärmeemissionsvermögen von weniger als 0,1, vorzugsweise weniger als 0,05, hat.

11. Flexible Abdeckung nach Anspruch 9 oder 10, wobei die wärmereflektierende Schicht auf der Abdeckung als eine Beschichtung bereitgestellt ist.

12. Flexible Abdeckung nach Anspruch 9 oder 10, wobei die wärmereflektierende Schicht die Abdeckung ist.

13. Flexible Abdeckung nach einem der vorhergehenden Ansprüche 9 - 12, wobei die Abdeckung eine wärmeisolierende Schicht aufweist, und wobei die wärmereflektierende Schicht auf einer Außenseite der wärmeisolierenden Schicht angeordnet ist.

14. Flexible Abdeckung nach einem der vorhergehenden Ansprüche 9 - 13, wobei die Abdeckung eine synthetische Membran aufweist, die zum Beispiel Polyvinylchlorid (PVC) aufweist, und wobei die wärmereflektierende Schicht auf einer Außenseite der synthetischen Membran oder gegebenenfalls auf einer Außenseite der wärmeisolierenden Schicht angeordnet ist.

## Revendications

1. Digesteur de biomasse, comprenant :
- un récipient pour contenir une biomasse et/ou un digestat de celle-ci, et
- un couvercle flexible pour recouvrir le récipient ;
**caractérisé en ce que**
ledit couvercle comprend une couche thermo-réfléchissante pour réfléchir le rayonnement émis par ladite biomasse et/ou ledit digestat.

2. Digesteur de biomasse selon la revendication 1, dans lequel ladite couche thermo-réfléchissante présente une émissivité thermique de moins de 0,1, de préférence de moins de 0,05.

3. Digesteur de biomasse selon la revendication 1 ou 2, dans lequel ladite couche thermo-réfléchissante est fournie sur ledit couvercle sous la forme d'un revêtement.

4. Digesteur de biomasse selon la revendication 1 ou 2, dans lequel ladite couche thermo-réfléchissante est ledit couvercle.

5. Digesteur de biomasse selon l'une quelconque des revendications précédentes, dans lequel ladite couche thermo-réfléchissante est agencée sur une face dudit couvercle qui est en face dudit récipient.

6. Digesteur de biomasse selon l'une quelconque des revendications précédentes, comprenant une pluralité de couvercles flexibles, dans lequel au moins un couvercle de ladite pluralité de couvercles comprend ladite couche thermo-réfléchissante.

7. Digesteur de biomasse selon l'une quelconque des revendications précédentes, dans lequel (a) ledit couvercle comprend une couche thermo-isolante, et dans lequel ladite couche thermo-réfléchissante est agencée sur une face externe de ladite couche thermo-isolante.

8. Digesteur de biomasse selon l'une quelconque des revendications précédentes, dans lequel (a) ledit couvercle comprend une membrane synthétique, par exemple comprenant un polychlorure de vinyle (PVC), et dans lequel ladite couche thermo-réfléchissante est agencée sur une face externe de ladite membrane synthétique, ou, le cas échéant, sur une face externe de ladite couche thermo-isolante.

9. Couvercle flexible agencé et destiné à recouvrir un récipient d'un digesteur de biomasse,
**caractérisé en ce que**
ledit couvercle comprend une couche thermo-réfléchissante pour réfléchir le rayonnement émis par une biomasse et/ou un digestat de celle-ci contenu dans ledit récipient.

10. Couvercle flexible selon la revendication 9, dans lequel ladite couche thermo-réfléchissante présente une émissivité thermique de moins de 0,1, de préférence de moins de 0,05.

11. Couvercle flexible selon la revendication 9 ou 10, dans lequel ladite couche thermo-réfléchissante est fournie sur ledit couvercle sous la forme d'un revêtement.

12. Couvercle flexible selon la revendication 9 ou 10, dans lequel ladite couche thermo-réfléchissante est ledit couvercle.

13. Couvercle flexible selon l'une quelconque des revendications 9 à 12, dans lequel ledit couvercle comprend une couche thermo-isolante, et dans lequel ladite couche thermo-réfléchissante est agencée sur une face externe de ladite couche thermo-isolante.

14. Couvercle flexible selon l'une quelconque des revendications 9 à 13, dans lequel ledit couvercle comprend une membrane synthétique, par exemple comprenant un polychlorure de vinyle (PVC), et dans lequel ladite couche thermo-réfléchissante est agencée sur une face externe de ladite membrane synthétique, ou, le cas échéant, sur une face externe de ladite couche thermo-isolante.
